# EUROPEAN PATENT APPLICATION

(11) **EP 1 044 716 A1**
(43) Date of publication of application: **18.10.2000**
(21) Application number: 99105222.6
(22) Date of filing: 13.03.1999
(51) Int. Cl.: B01D 57/02, G01N 27/447, C07K 1/28

(54) **Micropreparative isoelectric focussing**

(71) Applicant: Cahill, Michael, Dr., 72116 Moessingen (DE); Drukier, Andrzey, Dr., Burke, VA 22015 (US); Nordheim, Alfred, Prof. Dr., 72135 Dettenhausen (DE)
(72) Inventor: Cahill, Michael, Dr., 72116 Moessingen (DE); Drukier, Andrzey, Dr., Burke, VA 22015 (US); Nordheim, Alfred, Prof. Dr., 72135 Dettenhausen (DE)
(74) Representative: Patentanwälte Ruff, Beier und Partner

(57) **Abstract**

This invention provides a device for separation of mixtures of especially biological molecules into components by isoelectric focussing. The volume, in which electrophoretic separation takes places, is bordered by surfaces, which are at least partially coated with fixed buffer molecules, thus distinct pH barriers are defined. The device is further characterized by short distances between opposite surfaces, said surfaces are in particular parts of silica or plastic.

## Description

The invention relates to a device for separation of a mixture, especially a mixture of biological molecules, by isoelectric focussing according to the preamble of claim 1.

The use of electrophoresis for preparative purposes is an established technique and several types of electrophoretic apparatus exist for preparative and analytical purposes. These apparatus and their accompanying principles fall into four categories [A. T. Andrews, (1986). Electrophoresis: Theory, Techniques, and Biochemical and Clinical Applications, Clarendon Press, Oxford.]:
a) free flow electrophoresis
b) disc electrophoresis
c) isotachophoresis
d) isoelectric focussing

Both isotachophoresis and disc electrophoresis utilise hydrophilic matrices, and typically exhibit high resolution, but low loading capacities. Free flow electrophoresis utilises a continuously flowing thin buffer layer which enables high throughput. The method can be combined with isotachophoretic principles in interval isotachophoresis, by which a relative concentration sample specific fractions can be combined with high loading ability [Kasicka, (1994). J. Chromatogr. B. Biomed. Appl. 656: 99-106].

Isoelectric focussing has been performed in either liquid density gradients, in gel gradients, or in multicompartment apparatus with isoelectric immobiline based separating gel medium [P. G. Righetti et. al., (1997). J. Chromatogr. B. Biomed. Sci. Appl. 699: 105-115.]. Methods which rely on anticonvective separating hydrophilic gel media, with the exception of the latter, require post electrophoretic recovery of analyte molecules from within the gel matrix. These processes introduce the possibility of material loss during sample recovery, and also the risk of contamination from the gel medium. Both these parameters are critical when dealing with micropreparative sample amounts.

In devices utilising multicompartment isoelectric focussing, analyte molecules do not enter the gel matrix, but remain in buffer within a specific chamber while other molecules are removed by hydraulic flow and/or electrophoresis; passage through the restrictive gel being prevented by electrophoretic migration in the counter direction, the buffering values of walls on either side of the chamber being below and above of the pH value corresponding to the isoelectric point (pI) of the analytes retained. The porous filters of multicompartment isoelectric focussing apparatus are impregnated with a polyacrylamide gel matrix which contains immobilines of a defined pH buffering value. By placing such filters, each with a differing pH value, sequentially in a tube like vessel, an electrophoretic high pass/low pass pH barrier system was created whereby molecules were trapped between two such filters in an electrophoretic field when the pI of the molecule lies between the buffered pH of both filters. Note that in this prior art process the restrictive gel creates a continuous barrier across the electrophoretic path, with the buffer properties of the restrictive regions being determined by molecules based on immobiline chemistry.

By using multicompartment isoelectric focussing according to prior art technology the problem of material loss arises due to solid pH barriers, which are usually formed as gels or membranes. In particular this is important in the case of small sample volumes. The invention facilitates the task to provide a device for isoelectric focussing especially for small sample volumes, wherein material loss is minimized. This problem is solved by a device comprising the features of claim 1, preferred embodiments are shown in the following claims. The wording of all claims is hereby made to the content of the present specification by reference. The device according to the invention is described in greater detail below by way of example with reference to variants. For the sake of simplicity a major part of this disclosure describes the applications for the field of protein biochemistry; the generalization to other molecules, such as lipids or nucleic acids, will be possible for those skilled in the art.

In this disclosure the principles of isoelectric focussing will be used in an inventive manner whereby a series of fluid buffered pH barrier regions are created and the analyte molecules are induced electrophoretically to approach their pI and thus concentrate into regions which will be useful for their subsequent free flow fractionation. These regions are characterized by a low buffer capacity whereby molecules can be trapped between the high pass low pass pH barriers on either side. The disclosed device presents the analytes and buffer with a stratified and precisely defined medium, where within the array diverse molecules inducing different pK and buffering capacities are anchored or fixed, respectively. The pK of a buffering group is normally defined to be the pH where the buffering group is 50 % ionised, and where it exerts maximum buffering potential. The principle of the inventive device is based on the multicompartment isoelectric focussing, wherein instead of the known solid pH barriers fluid pH barriers, especially planar fluid pH barriers are formed, which allow a continous solution fluid and which minimize sample material loss.

The wording of all claims is incorporated in this description by reference.

Said pH barriers are formed by buffer molecules, which are fixed on surfaces bordering the electrophoretic volume. By the word "fixed" is meant, that the buffer molecules are bound or ligated to said surfaces forming a coated or semi-coated surface, wherein the immobilisation of buffering molecules according to the state of the art within a gel matrix is excluded. The buffering molecules could be immobilines covalently attached to the surface of the electrophoresis chamber without the presence of polyacrylamide, or other such buffering molecules, such as modified amino acids or oligo-peptides, or indeed any molecule containing ionisable groups.

The electrophoretic (three dimensional) volume, especially formed as a channel-like chamber, is encased by two juxtaposed, especially opposite surfaces, broken into homogenous regions which are coated with molecules possessing a certain conductivity and buffer capacity at a given pH. One or both said surfaces may comprise a coating of buffering molecules. The said surfaces of the inventive device are for example the surfaces of two opposite silica or plastic chips, not truly planar or (especially) planar, with certain opposite regions being coated with molecules which impart defined buffering and conductivity properties to aqueous buffer within the electrophoretic volume between said surfaces. By "chip" is meant a micro-apparatus of the type where electrical circuitry or microfluidic channels can be created by modern methods such as photoetching, and which will be apparent to those expert in the art. Between said surfaces there is no contact other than at the edges of the surfaces, where the volume or chamber is physically sealed by non buffering adhesives and/or physical spacers. The distance between said surfaces is typically between 10 and 1000 nm, especially between 10 and 500 nm. Extremely small distances between two planar surfaces can be maintained by columns of approximately 10 to 30 nm diameter which are created when the surrounded surface is removed by microetching. Such columns occupy a negligible volume relative to the volume of that region of the electrophoretic volume or chamber, and exert negligible effects on electrical conductivity, fluid flow and micro-turbulences. In preferred embodiments of the invention the generation of these extremely small distances permits the pH of the aqueous medium to be determined by buffering molecules immobilised on the surface of the chamber.

The miniaturisation of isoelectric focussing permits the creation of restrictive buffer regions somewhat similar to those generated in multicompartment isoelectric focussing apparatus. However the device disclosed differs in two critical respects. Due to the miniaturisation obtained, the restrictive medium does not physically span the electrophoretic volume or chamber from wall to wall. Indeed a direct and unbroken buffer channel exists between all regions of the device. This is possible since the microscale allows buffer molecules on the walls of the electrophoretic volume itself to determine the pH of the buffer within. The buffer molecules need not to be immobilines, which is the second important difference to the known multicompartment isoelectric focussing apparatus. Instead they consist especially of a skeleton or backbone chain of hydrocarbon- or fluorocarbon-based molecules with terminal buffer groups which have been attached, especially by covalent bonding, to the surface of preferentially a chip by e.g. photoetching, derivatisation methods, micro-contact printing, self assembly monolayer reactions, or other prior art methods. By self assembly monolayer is meant a system where a surface is functionalized to allow covalent bonding to one or more types of specific substrate molecules, such that the surface binds substrate in a specific manner, in the ideal case forming a closely packed monolayer.

An innovative multi-channel fluidic system according to the invention is formed by high spatial resolution dense patterns of grooves and miniaturised vessels, provided by e.g. modern photoetching methods, especially applicable to silica but not restricted thereto, and electric field activated and/or computer controlled fluidic switches. Such fluidic systems can be produced not only in silicon dioxide, but also in plastic, especially in teflon, and a plurality of other solid state materials. It is pointed out that for the proposed application the main challenge besides the channel's definition is mainly the quality of surface which should be prepared so as to make negligible the adherance of components from heterogenous mixtures of molecules, such as proteins. In this context teflon as material, especially as coating material, for the fluidic system is especially preferred, because due to the nature of this material hydrophobic interactions with the sample material are as far as possible excluded and thereby material loss is minimized. The body of the device could be made of one material, e.g. (but not restricted to) silica, tungsten, suitable nitrides and carbides, diamond, or plastic, while the surface to which buffering and conducting molecules are attached, and which is in contact with analyte molecules during electrophoresis, could be made of another material, e.g. (but not restricted to) teflon.

Due to Joule heating, temperature control is advisable to diminish the probability of thermally induced flow instabilities and turbulences. For this purpose solid state materials with very high thermal conductivity such as nitride, carbide, or diamond filled teflon are preferred. Furthermore, because of the very small diameter of these channels, say below 10 microns, flow homogeneity is an issue because any roughness of the channel surface may lead to turbulences and thus will make separation unreliable. On the other hand, use of viscous fillers diminishes the turbulence probability, both for sheer flow and thermally induced turbulence. However, even taking into account these limitations, the "in-silico" implementation of multi element fluidic systems is justified by their much higher packing density and better performance than hybrid systems. By hybrid system is meant a system in which separate systems such as microcapillaries of HPLC (high performance liquid chromatography) columns are connected through a series of external pipings.

The use of in-silico fluidic systems permits handling of very small sample volumes. If in the classical capillary systems up to 500 nl of fluid has to be loaded, in the in-silico systems only a few tens of nl can be loaded. This draws attention to the detection method, because often sub fmol amounts of the material of interest are to be detected. Thus the use of maximum sensitivity detectors coupled with the in-silico fluidics is disclosed as preferred implementation of the proposed method of fractionation of small sample volumes. In particular, a preferred implementation involves an array of miniaturised prior art isotope detectors such as scintillation detectors or Multiple Photon Detection (MPD) detectors.

MPD (e.g. US Patent Number 5532122) is a technique for the detection and measurement of certain radioisotopes at activity levels which are much below the naturally occurring background. This is made possible by the nearly perfect rejection of background events which is achieved through sophisticated signal processing and analysis. Certain isotopes emit both X-rays as electrons move to lower orbitals, and gamma rays as neutrons decompose upon radioactive decay. The high degree of sensitivity of MPD is obtained by considering only signals with coincident X- and gamma-rays, and where the energy profile of the measured photons corresponds to those expected for the isotope in question. The isotopes compatible with MPD come from two broad families of radioisotopes which together include over 100 members appropriate for use as tags. In particular, they include the isotope iodine-125 (¹²⁵I), one of the most commonly used isotopes in biomedical diagnostics. By carefully comparing each detected event with the highly specific decay signature of the used radioisotope, MPD is able to reduce the number of background events to less than one per week. MPD permits multi-labeling (up to 16 co-resident labels), good spatial resolution (about 0.3 mm) and very high dynamic range (linearity over nine orders of magnitude in label down to 10⁻²¹ mole). Two significant advantages which are derived from this capability are that substances labeled with appropriate isotopes can be reliably detected at four orders of magnitude lower concentrations, and the amount of isotope which must be used is reduced 1,000-fold to a level much below the naturally occurring background radiation.

One advantage of the very small dimensions of in-silico fluidic systems is that large electrical field strengths can be applied, typically 3 to 50 kV, especially 5 to 40 kV, and more especially 10 to 20 kV. Consequently, individual separations are carried out very quickly. In one embodiment of the device the electric fields can be varied with time, strength, or patterns thereof. Because of the short analysis times required, the disclosed device is used in a quasi continuous cycle, thus increasing the effective load capacity.

The principles of operation of the device according to the enclosed invention will also be explained with reference to the following figures:
- Figure 1:: first embodiment of the inventive device (called pH multiplexer)
- Figure 2:: section along the longitudinal axis of the device of Fig. 1
- Figure 3:: further embodiment of the inventive device as a tree-like series

The scope of this invention is not limited to the disclosure of these figures. All features discussed in the following, separately or in combination, are part of the invention.

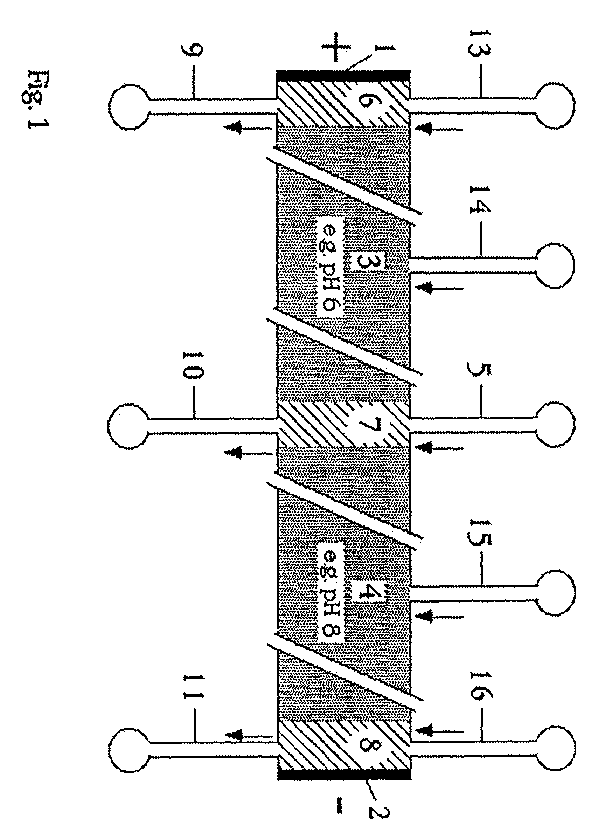

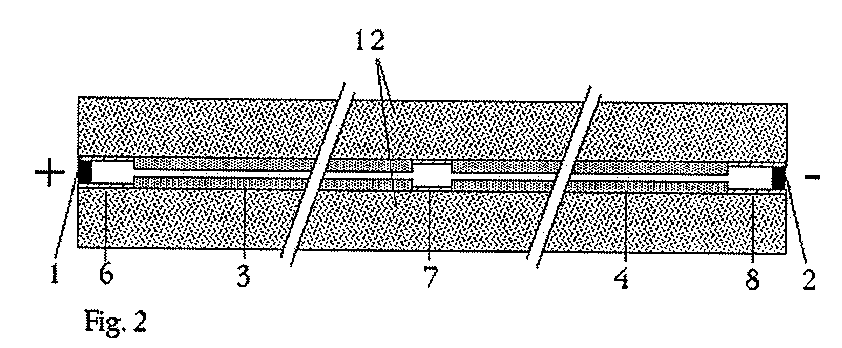

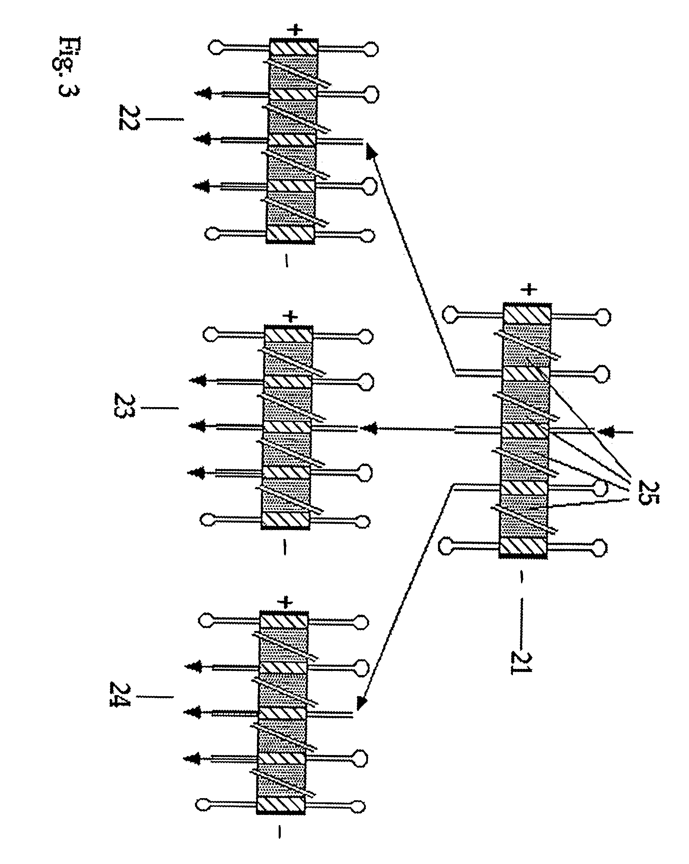

One implementation of the device according to the present invention is presented in Fig. 1 and 2. This pattern, and variations thereof, like in Fig. 3 (but not restricted thereto), define the arena in which separations according to this principle and device, and other variations thereof, will be performed.

A device according to the invention as shown in Fig. 1 and 2, consists of an electrophoretic volume, here formed as a channel-like chamber (no reference number), with anode 1 at the end of the most extreme acid pH buffered barrier, and cathode 2 at the opposite end of the chamber with the most extreme basic pH buffered barrier. In this particular implementation the channel-like chamber has a very small dimension, the distance between the opposite surfaces 12 is especially less than 10 µm and may be as small as 500 nm to 10 nm. The length of the chamber is shown in a shortened manner indicated by the diagonal lines (obliques), which are used in all figures. Distinct pH barriers 3 and 4, which are built up by buffer molecules fixed to the two opposite surfaces 12 or to surfaces attached to these surfaces 12 bordering the electrophoretic chamber, subdivide the chamber into regions with defined pH value, called barrier regions, e.g. pH 6 in region 3 and pH 8 in region 4, and collection regions 6, 7, and 8 surrounding the pH barriers. Inlet channels 5, 13, 14, 15, and 16 lead towards the electrophoretic chamber or the corresponding regions, respectively, and outlet channels 9, 10, and 11, lead away.

Analyte molecules are induced to migrate electrophoretically or are carried by bulk fluid movement through the system. The electrophoretic chamber contains pH barrier regions 3 and 4 of buffered pH, e.g. pH 6 and pH 8 in Fig. 1, which define the charge and therefore the electrophoretic migration of the analytes. Voltage applied across the isoelectric focussing electrodes 1 and 2 results in electophoresis of the analytes towards their isoelectric point.

In the presence of an electric field between electrodes 1 and 2, analytes migrate until they arrive at a region where they are close to being neutrally charged, such that movement in either direction requires traversing an impassable electrophoretic barrier, e.g. a molecule with pI of 7 entering the chamber from channel 14 into pH barrier 3 (pH 6) is positively charged and migrates towards cathode 2. When it moves from pH barrier 3 (pH 6) into collection region 7, it could not successfully re-enter pH barrier 3 (pH 6) since at this pH the molecule would be negatively charged and migrate towards cathode 2. Likewise, if the molecule migrates into pH barrier 4 (pH 8) it would become negatively charged and migrate towards anode 1. Thus molecules will be effectively trapped between high pass and low pass barriers because the pI of each molecule lies between the pH of the two surrounding buffer barriers. After having accumulated in the collection region, e.g. 7, the molecule can be induced to leave the electrophoretic chamber via e.g. introduction of hydraulic flow from the corresponding outlet channel 10.

In the implementation of the device depicted in Fig. 1 and 2 there are two such pH barriers 3 and 4, with buffered pH values of pH 6 and pH 8, respectively. However many such barriers and collection regions could be incorporated in other manifestations of the device. Upon completion of the isoelectric focussing-like step, these barriers impose a pH multiplex function upon the analyte molecules in the sample such that they are divided into fractions in collection regions 6, 7 and 8, where the isoelectric point of trapped analytes have the distribution pI < 6 in collection region 6, 6 < pI < 8 in collection region 7, and pI > 8 in collection region 8, respectively. Analyte molecules from collection region 6 can be removed via an outlet channel 9. Analyte molecules from collection regions 7 and 8 could be removed via channels 10 and 11 respectively, experimental design permitting. The channels referred to here can be capillaries, but could be any vessel, pipe, tube or microchannel permitting (preferably controlled) fluid flow, including microchannels on a chip or a chip based fluid filter. All channels can be connected to receptacles/ reservoirs, graphically indicated in Fig. 1 as circular entities, but not designated by reference numbers.

In a preferred embodiment of the present invention fluid is introduced to the electrophoretic volume, realized as a channel-like chamber, by hydraulic force from channel 13 at the anode or channel 16 at the cathode region, and/or by channels 14 and 15 which discharge into buffered regions 3 and 4 respectively, and/or by channel 5 which discharges into collection region 7. Fluid can be removed from the electrophoretic chamber via channels 9 and 11 after the fluid has been induced to traverse at least part of the chamber. Note that if fluid was introduced to the electrophoretic chamber via channel 13 or via channel 14, it would normally be induced to exit the chamber via channel 11. The same would apply for entry from channels 16 or 15, with exit via channel 9. If fluid entered via channel 5 it could be induced to flow out of the chamber via channels 9 and/or 11, as desired. The required fluid pumping force could be supplied by an external mechanical or electroendoosmotic pump, whereby fluid hydrostatic cohesion would pull fluid, containing analytes, through the whole device as described above. The transport of fluids containing analytes is achieved either with the aid of pumps and valves, and/or by the application of electric fields to suitable portions of the system. Note that for fluid pumping by an electroosmotic pump it may be advantageous to alternate between an electric field across 1 and 2 which induces isoelectric focussing between buffer regions, and one or more electric fields which introduce and expel fluids from the system at one or both ends, e.g. from channels 13 to 11 or from 16 to 9.

In a preferred embodiment of the invention an electric field transporting analytes into the electrophoretic volume or chamber is applied in addition to the external pumping force, e.g. a mechanical or electroendoosmotic pump. This embodiment provides the important advantage, that the analytes are charged at the moment of entering the electrophoretic volume or chamber. Hereby the isoelectric focussing of analytes starts instantly and is much faster, resulting in a shortened separation time compared to apparatus of the prior art. In a particular implementation of the inventive device the device can be transversed by fluid flow in a controlled manner from the inlet and outlet sources concurrently with isoelectric focussing rather than intermittently.

By use of valves and altered application of electrical fields, fluids could be introduced from channels 13 or 16 leading from a reservoir containing analyte molecules at a given end, and also removed from the same end via channels 9 or 11. This fluid removal system facilitates removal of small, highly mobile ions which originate from the sample. By appropriate application of voltage between different electrodes these ions are removed from the plane of analysis of less mobile analytes, thus reducing joule heating and allowing subsequent application of higher voltages across the region of the array containing analyte molecules, and thereby decreasing separation time.

In a preferred embodiment of the inventive device the fluid flow through the inlet channels and outlet channels renews the electrolyte solutions and sample buffer in specified regions to the electrophoretic volume or chamber, such as the cathodic and anodic zones.

The described process reduces the complexity of the analysed sample within a particular fraction, while increasing the concentration, and thereby improves the ability to detect low abundance components in a concentration dependant manner in this and subsequent analyses. The device provides additionally the advantage that migration through a channel or capillary during subsequent analysis will not be perturbed by overloading leading to difficulties in controlling artifacts. This reduction of complexity in order to facilitate subsequent analysis of complex mixtures of molecules is also innovative when applied to such a pH multiplexer. It is envisaged that electroosmotic flow will be experienced at extreme pH values. However this effect will be less than that observed at similar pH ranges in IPG (immobilised pH gradient) gels since the composition of the array at any one point is defined only by the buffer requirements. This is in contrast to IPGs where there exist many charged buffer species which are not involved in the actual pH titration at that pH. For instance at pH 3 in an IPG the acrylamide matrix contains certain defined concentrations of amino groups which are fully protonated at that pH, but which were important for buffering at higher pH values and thus were included in the gel mixture. The presence of these charged molecules induces electroendoosmosis in IPGs, by which e.g. immobilised anions are unable to migrate electrophoretically to the cathode, and instead induce a bulk fluid flow towards the anode. Such effects will be minimised in the disclosed device, and could be compensated for by removal or replacement of fluid at an appropriate rate from the capillary pumps described above.

After analyte molecules have been concentrated in the collection regions between pH barriers, they are removed by the aid of further channels which induce fluid flow in another direction to the isoelectric fractionation by use of hydraulic forces, or by the application of electric fields to suitable portions of the system as described above. These channels lead simply to a collection vessel (reservoir), or preferentially are themselves part of a further fractionation device such as microbore HPLC (high performance liquid chromatography) or CE (capillary electrophoresis). In preferred variants of this embodiment the outlet channels are connected to subsequent further fractionation steps, such as discrimination by density sedimentation, discrimination by particle size, melting point, mechanical properties, polarity (electric moments), isoelectric point, affinity, spectroscopic properties, chromatography or electrophoresis.

In a preferred embodiment of this invention the electrophoretic volume is fitted with e.g. at least one of the following devices: a device for keeping the electrophoretic volume at a controlled temperature, a device for pH measurement and control, a device for conductivity monitoring, a biosensors detection system, an immunoelectrophoretic device, a laser excited fluorescence detection system, a device for radioisotop monitoring, preferably a MPD detection system, a robotically coupled system, a device for monitoring the sample solution in the UV or visible light or by fluorescence observation, or a device for measuring the dielectric constant of the sample solution.

The inventive immobilised buffer array uses immobilised buffering molecules. According to the invention there is no direct physical contact of molecules from one wall of the electrophoretic volume or chamber with molecules on the other wall, with the resulting uninterrupted buffer channel fundamentally distinguishing this device from prior art multichamber isoelectrophoresis apparatus. Any buffering molecule in principle can fulfill the buffering function, and indeed will be preferable without the structural and chemical constraints imposed by the acrylamide-like backbone of immobilines. In a preferred variant of the invention the array is created using suitable precursors and microcontact printing, self assembly reactions, or photo-activated chip technology where reactions take place in photo-activated regions, or other prior art methods. However the principle and device variants would be functional over a large range of physical dimensions.

The process of protein separation obtained by this device is similar to free flow isotachophoresis in result, however the separating molecules are not soluble ions, but immobilised buffer molecules. Thereby the point of exit of analytes from the array is strictly defined, and not sensitive to slight ionic buffer variations, as is the case in isotachophoresis. Thus the concept and device here disclosed are novel and innovative.

In a further variant of the device described above, the outlet channels 9, 10, or 11 of the multiplex may be connected to a receptacle tube which could provide the required pump action.

In a particular implementation of the invention occuring air bubbles in the electrophoretic volume or chamber are removed by hydraulic flow and/or by degassing solutions under vacuum before application to the device.

In a further variant of the inventive device several pH multiplex regions are incorporated, such that analyte molecules are partitioned into several different analytical compartments.

A preferred embodiment of the invention arranges several such pH multiplexers in a tree-like series, one example of which is depicted in Fig. 3. The possible number of arrangements of such configurations is unlimited, in principle. First the components of the sample are fractionated in a pH multiplexer 21 as essentially described above, wherein the pH barriers 25 are chosen out of a relatively broad range, e.g. pH 3, pH 5, pH 7, and pH 9. Following the first fractionation, the components are introduced to further pH multiplexers 22, 23, and 24, wherein pH definition is more narrow, and thereby a much more precise fractionation is possible, e.g. a fractionation of components, which differ in their isolelectric points by at least 0.05 pH units.

In a further embodiment of all inventive devices the electrophoretic volume or chamber is filled with solutions of varying viscosity. The volume or chamber is attached to a centrifugal device capable of generating gravitational force along the one axis. Thereby analyte molecules are separated by isoelectric pH multiplex properties along one axis, and by density along another axis. The density separation is used as a further concentration method if proteins are loaded between two density cushions in a step gradient.

Because of the multiplex effect obtained, the inventive devices obtain a fine division of incoming fluid into collection regions with very narrow pH definition. In any filtering system the losses increase with depth. Thus it is crucial that any and all low/high pass pH filters are ideally lossless, i.e. they implement a step function in pH. Creation of a true pH step gradient at the delta region, the amplitude of the buffering capacity, and the voltage applied over the delta region are critical in this respect. Using a device on the scale of a microchip according to the invention, large voltages can be applied, being advantageous to minimise losses. Critical can be maintenance of solubility and control of field strength with respect to temperature, which determines the time available for molecules to electrophoretically leave the delta region. It cannot be excluded that some small but real number analytes may be trapped by the pH barriers. The inventive solution to this problem involves duplicate analysis of samples by complementary pH multiplexes, such that for instance one multiplex separates analytes between pH regions 3-7, and 7-11, while a second complementary multiplex separates identical analytes between pH 5-9.

Another preferred application of the disclosed multiplex pH system is its use in combination with a coupled parallel HPLC/CE separation. Here the benefits of each technique are non-overlapping, thus an optimal reduction of complexity of the analytes is achieved. Further, the pH barriers chosen in the array determine the optimum IPG gel into which HPLC/CE sorted analytes are delivered.

The practical limitation of two dimensional (2D) gel systems is a relatively low throughput and a need for a very powerful pattern recognition software to properly identify each spot. There are biomedical applications in which the total number of proteins is higher than the resolution limit of CE (500-1,000 proteins) and lower than a maximum resolution of optimised 2D gels (up to 10,000 proteins). For a few thousand proteins the above proposed system is an appropriate high throughput/low cost solution. Another preferred embodiment of the subject invention is an innovative and optimised device for reduction of sample complexity which thereby should facilitate analysis of the full dynamic range of analytes. If e.g. in the device according to Fig. 3 the last level of the tree is controlled by an appropriate high sensitivity detector, the device is able to discriminate which fractions are high and low abundance. If the total flow through of a given channel of the above described pH multiplex is higher than a computer established threshold, an appropriate pulse is sent, and the outlet of this channel is closed. Thus the selection of only the pH channels for which the amount of proteins or detected entities (e.g. radioactively labelled amino acids) is lower or higher than a desired value is achieved in parallel. Thus this device is used to separate by abundance additionally to pI, as described in this disclosure. An inherent advantage of this separation device is that the pH interval of a subsequent IPG required to analyse the resulting fractions by 2D gels would be automatically defined by the nature of the pH multiplex applied.

## Claims

1. Device for separation of a mixture, especially a mixture of biological molecules, into components by isoelectric focussing, said device having surfaces at least partially bordering at least one (three dimensional) volume in which such electrophoretic separation takes place,
characterized in that buffer molecules are fixed on at least a part of said surfaces.

2. Device according to claim 1, characterized in that the volume is a chamber, especially a channel-like chamber.

3. Device according to claim 1 or 2, characterized in that on the surfaces first regions with fixed buffer molecules and second regions without fixed buffer molecules and/or with fixed buffer molecules imparting lower buffering capacity are provided, said first and second regions defining pH barrier regions (3, 4) and collection regions (6, 7, 8), respectively.

4. Device according to claim 3, characterized in that pH barrier regions and collection regions are provided in an alternating sequence.

5. Device according to one of claims 3 or 4, characterized in that at least two pH barrier regions are provided.

6. Device according to one of the preceding claims, characterized in that said surfaces are arranged opposite to each other, wherein preferably the surfaces are arranged at least partially in a short distance opposite to each other, wherein preferably the distance between the surfaces is between 5 nm and 100 µm, especially between 10 nm and 1 µm.

7. Device according to claim 6, characterized in that pH barrier regions and collection regions are formed by fixing or non-fixing buffer molecules, respectively, on opposite surfaces or parts of opposite surfaces, wherein in particular the same kind of buffer molecules are fixed on opposite surfaces or parts of surfaces for forming a specific pH barrier region.

8. Device according to one of the preceding claims, characterized in that the surfaces are surfaces of parts of tungsten, a nitride, a carbide, diamond, silica, plastic, especially teflon, and/or surfaces of parts coated with such materials.

9. Device according to one of the preceding claims, especially according to claim 8, characterized in that the surfaces are surfaces of parts, which are in the form of chips.

10. Device according to one of the preceding claims, characterized in that the buffer molecules are fixed by bonding, especially covalent bonding.

11. Device according to one of the preceding claims, characterized in that the buffer molecules are fixed using photoetching methods, micro-contact printing, self assembly monolayer reaction, and/or other derivatisation methods.

12. Device according to one of the preceding claims, characterized in that the buffer molecules comprise a hydrocarbon- or fluorocarbon-based skeleton or backbone, wherein said buffer molecules especially are fixed to the surfaces via said skeleton or backbone.

13. Device according to one of the preceding claims, characterized in that the buffer molecules comprise terminal and/or internal buffer groups.

14. Device according to one of the preceding claims, further characterized in that the volume, in which the electrophoretic separation takes place, is provided with several vessels, especially with at least one inlet pipe or inlet channel (5, 13 to 16) connected to at least one pH barrier region (3, 4) and/or to at least one collection region (6, 7, 8) and at least one outlet pipe or outlet channel (9, 10, 11) connected to at least one pH barrier region and/or at least one collection region (6, 7, 8), wherein preferably at least some of the inlet pipes or inlet channels and of the outlet pipes or outlet channels are connected to receptacles or reservoirs.

15. Device according to one of the preceding claims, characterized in that it is designed for applying a hydraulic and/or a electric force to the mixture, especially for introducing the mixture to said volume, in which the electrophoretic separation takes place, said device in particular comprising a mechanical or endoosmotic pump and/or at least one device for generation of at least one electric field in addition to the electric field for the isoelectric focussing.

16. Device according to one of the preceding claims, characterized in that it is designed for applying electric voltages from 3 kV up to 50 kV, especially 10 to 20 kV, to said volume for said isoelectric focussing.

17. Device according to one of the preceding claims, characterized in that the mixture contains peptides and/or proteins.

18. Device according to one of the preceding claims, characterized in that molecules of the mixture, especially peptides and/or proteins, are at least partially derivatized, especially isotopically derivatized, to allow detection of these molecules.

19. Device according to one of the preceding claims, characterized in that the mixture is an aqueous solution, especially a buffered aqueous solution.

20. Device according to one of the preceding claims, characterized by being coupled with or to at least one device for isotopic detection, especially a scintillation device or MPD detector.
